# EUROPÄISCHE PATENTSCHRIFT

(11) **EP 3 503 936 B1**
(45) Veröffentlichungstag und Bekanntmachung des Hinweises auf die Patenterteilung: **29.09.2021**
(21) Anmeldenummer: 17749722.9
(22) Anmeldetag: 09.08.2017
(51) Int. Cl.: A61M 1/00

(54) **VORRICHTUNG ZUR UNTERDRUCKBEHANDLUNG VON WUNDEN AM MENSCHLICHEN KÖRPER**
DEVICE FOR THE VACUUM TREATMENT OF WOUNDS ON THE HUMAN BODY
DISPOSITIF DE TRAITEMENT PAR PRESSION NÉGATIVE DE PLAIES AFFECTANT LE CORPS D'UNE PERSONNE

(30) Priorität: 25.08.2016 DE 102016115835
(43) Veröffentlichungstag der Anmeldung: 03.07.2019
(73) Patentinhaber: Paul Hartmann AG, 89522 Heidenheim (DE)
(72) Erfinder: HOFSTETTER, Juergen, 52349 Düren (DE); BEYRLE, Karina, 89075 Ulm (DE); MOTTSCHELLER, Markus, 90489 Nuernberg (DE); KLEIN, Peter, 71254 Ditzingen (DE); MURGULESCU, Mihai, 90411 Nuernberg (DE)
(74) Vertreter: DREISS Patentanwälte PartG mbB
(86) Internationale Anmeldenummer: PCT/EP2017/070206
(87) Internationale Veröffentlichungsnummer: WO 2018/036823

(56) Entgegenhaltungen:
- WO-A1-2009/004371
- DE-A1-102014 226 890
- GB-A- 2 307 180
- US-A1- 2009 240 218
- US-A1- 2011 106 028
- US-A1- 2013 267 918
- US-B1- 7 611 500

## Beschreibung

Die Erfindung betrifft eine Vorrichtung zur Bereitstellung von Unterdruck zur Unterdruckbehandlung von Wunden am menschlichen Körper, mit einer Unterdruck erzeugenden Einrichtung und einem Behälter zur Aufnahme von Flüssigkeiten in einem Inneren des Behälters, insbesondere von aus einer Wunde abgesaugten Wundsekreten, mit einem Anschluss für eine zum Körper führende Saugleitung, wobei die Unterdruck erzeugende Einrichtung in einem ersten Gehäuseteil der Vorrichtung angeordnet ist und der Behälter von einem zweiten Gehäuseteil der Vorrichtung gebildet ist, wobei der zweite Gehäuseteil der Vorrichtung an dem ersten Gehäuseteil befestigbar und manuell lösbar ist und im befestigten Zustand das Innere des Behälters über einen Anschluss von der Unterdruck erzeugenden Einrichtung mit Unterdruck beaufschlagbar ist, so dass eine Unterdruckkommunikation zwischen der Unterdruck erzeugenden Einrichtung, dem Behälter und der zum Körper führenden Saugleitung herstellbar ist, wobei wenigstens ein Gehäuseteil in einer Seitenansicht im Wesentlichen L-förmig ausgebildet ist.

Vorrichtungen zur Unterdruckwundbehandlung sind bereits mehrfach aus dem Stand der Technik bekannt. Eine Vorrichtung der vorausgehend genannten Art ist bekannt aus US 2013/0267918 A1.

Bei derartigen Vorrichtungen zur Unterdruckbehandlung von Wunden kommuniziert eine Unterdruck erzeugende Einrichtung über eine Saugleitung mit der Wunde oder der Wundumgebung, wobei ein luftundurchlässiges Abdeckmaterial zum luftdichten Verschließen der Wunde und der Wundumgebung vorgesehen ist, so dass ein Unterdruck im Wundraum herstellbar ist und Flüssigkeiten aus dem Wundraum in den genannten Behälter absaugbar sind.

Der Begriff eines Unterdrucks bezeichnet im Zusammenhang mit der vorliegenden Erfindung einen gegenüber dem Umgebungsluftdruck (atmosphärischer Luftdruck) erniedrigten Luftdruck insbesondere innerhalb eines Wundverbands. Das Abdeckmaterial eines Wundverbands zum luftdichten Verschließen eines Wundraums muss daher so ausgebildet sein, dass es der sich einstellenden Druckdifferenz standzuhalten vermag, damit der Unterdruck im Wundraum überhaupt angelegt und aufrechterhalten werden kann.

Im Bereich der Unterdrucktherapie in der Wundbehandlung wird der Unterdruck quantitativ als Druckdifferenz zwischen dem Umgebungsluftdruck und dem unterhalb des Abdeckmaterials angelegten Luftdruck angegeben. Typischerweise beträgt diese Druckdifferenz im Bereich der Unterdrucktherapie höchstens 250 mm Hg (mm Quecksilbersäule) (1 mm Hg = 1 Torr. = 133,322 Pa). Dieser Unterdruckbereich bis höchstens 250 mm Hg hat sich als für die Wundheilung geeignet erwiesen. Ein bevorzugter Unterdruckbereich liegt zwischen 10 und 150 mm Hg.

Der unter Verwendung der Vorrichtung an die Wunde angelegte Unterdruck kann bei einer typischen Unterdruckbehandlung entweder zeitlich im Wesentlichen konstant gehalten werden, oder er kann zeitlich verändert, insbesondere zyklisch verändert werden, was über eine entsprechend ausgebildete und programmierte Steuervorrichtung bei der Unterdruck erzeugenden Einrichtung, insbesondere in Abhängigkeit weiterer Parameter, realisiert werden kann.

Zum Anlegen von Unterdruck und vorzugsweise auch zum Absaugen von Körperflüssigkeiten ist eine vorzugsweise flexible Saugleitung, beispielsweise in Form eines Drainageschlauchs, vorgesehen, der einenends über einen sogenannten Port im Bereich des Wundabdeckmaterials mit der Wundumgebung oder dem Wundraum und anderenends mit dem eingangs erwähnten Behälter zur Aufnahme von Körperflüssigkeiten bzw. mit der Unterdruck erzeugenden Einrichtung kommuniziert.

Der erste und der zweite Gehäuseteil sind, insbesondere zum Wechseln des Behälters, welcher durch den zweiten Gehäuseteil gebildet wird, manuell lösbar aneinander fixierbar.

Der vorliegenden Erfindung liegt die Aufgabe zugrunde, für eine Vorrichtung der vorausgehend beschriebenen Art die Befestigung der Gehäuseteile aneinander zu verbessern, so dass diese zum einen auf sichere Art und Weise aneinander befestigbar sind und zum anderen die Handhabbarkeit der Vorrichtung komfortabel und benutzerfreundlich ist.

Diese Aufgabe wird erfindungsgemäß gelöst durch eine Vorrichtung mit den Merkmalen des Anspruchs 1.

Im Sinne der vorliegenden Erfindung ist mit einer L-Form gemeint, dass zwei Schenkel oder zwei Teile eines Gehäuseteils unter einem Winkel von etwa 85° bis 95°, vorzugsweise 90° zusammenlaufen oder zueinander erstreckt sind, wobei die beiden Schenkel oder Teile des Gehäuseteils eine gleich große oder eine unterschiedlich große Erstreckung aufweisen können. Dabei ist es denkbar, dass die L-Form dadurch ausgebildet ist, dass ein Teil bzw. ein Schenkel eines Gehäuseteils durch Anfügen eines Teils bzw. eines Schenkels zu einer L-Form ergänzt wird. Vorzugsweise ist die L-Form des Gehäuseteils dadurch ausgebildet, dass sich ausgehend von einem Teil bzw. Schenkel des Gehäuseteils ein weiterer Teil bzw. Schenkel vorspringend erstreckt.

Der L-förmige Gehäuseteil bildet eine Schiebesitzführung für den anderen Gehäuseteil, so dass der andere Gehäuseteil über die Schiebesitzführung an den L-förmigen Gehäuseteil angefügt werden kann, indem der andere Gehäuseteil geführt auf den L-förmigen Gehäuseteil aufschiebbar ist. Vorzugsweise wird, wenn sich die Gehäuseteile in dem bestimmungsgemäß gefügten Zustand befinden, automatisch eine Unterdruckkommunikation zwischen dem Inneren des Behälters des zweiten Gehäuseteils und der Unterdruck erzeugenden Einrichtung des ersten Gehäuseteils hergestellt.

Erfindungsgemäß ist vorgesehen, dass auf einer Innenseite eines Schenkels des L-förmigen Gehäuseteils wenigstens ein erstes Schiebeführungsmittel ausgebildet ist, und der andere Gehäuseteil ein zu dem ersten Schiebeführungsmittel komplementäres zweites Schiebeführungsmittel umfasst, so dass der andere Gehäuseteil auf den Schenkel des L-förmigen Gehäuseteils translatorisch geführt aufschiebbar ist, wobei die Schiebeführungsmittel ineinander eingreifen. Werden die ersten und zweiten komplementär zueinander ausgebildeten Schiebeführungsmittel miteinander in Eingriff gebracht, können die beiden Gehäuseteile durch eine translatorische Bewegung in der Schieberichtung geführt in ihren bestimmungsgemäß gefügten Zustand gebracht werden.

Erfindungsgemäß umfassen das erste und das zweite Schiebeführungsmittel wenigstens eine Führungsschiene und wenigstens eine dazu komplementäre Führungsnut.

Vorzugsweise ist der Schenkel des L-förmigen Gehäuseteils durch eine Schenkelplatte gebildet, die insbesondere eine Dicke quer zu ihrer flächenhaften Erstreckung von höchstens 10mm, insbesondere höchstens 8mm, insbesondere höchstens 5mm, insbesondere höchstens 3mm, aufweist. Mit einer Schenkelplatte ist im Sinne der vorliegenden Erfindung gemeint, dass der Schenkel eine plattenartige Gestalt aufweist, wobei die Erstreckung in zwei orthogonal zueinander stehenden Richtungen größer ist, als die Erstreckung in einer orthogonal zu den ersten zwei Richtungen ausgebildeten dritten Richtung.

Es erweist sich als vorteilhaft, dass in dem bestimmungsgemäß gefügten Zustand der zweite Gehäuseteil mit dem ersten Gehäuseteil auf Stoß liegt. Hierdurch wird vermieden, dass zwischen den beiden Gehäuseteilen insbesondere Schmutz aufnehmende Fugen, Ritzen oder Spalten gebildet werden.

In weitergehender Ausbildung der Erfindung erweist es sich als vorteilhaft, dass der erste Gehäuseteil in der Seitenansicht im Wesentlichen L-förmig ausgebildet ist und der zweite Gehäuseteil ein den ersten Gehäuseteil komplementär ergänzendes Profil aufweist, so dass die Vorrichtung im Wesentlichen eine Scheibenform aufweist, wenn sich die Gehäuseteile in dem bestimmungsgemäß gefügten Zustand befinden. Vorzugsweise ist der erste Gehäuseteil derart ausgebildet, dass die Unterdruck erzeugende Einrichtung in einem Teil des ersten Gehäuseteils angeordnet ist und sich ausgehend von diesem Teil eine Wandung des ersten Gehäuseteils plattenartig vorspringend erstreckt, wobei diese plattenartig vorspringende Wandung die oben erwähnte Schenkelplatte bilden kann und der erste Gehäuseteil auf diese Weise ein in der Seitenansicht betrachtet L-förmiges Profil aufweist. Vorzugsweise ist der zweite Gehäuseteil komplementär zu dem L-förmigen Profil insbesondere quader- oder scheibenförmig ausgebildet. Die scheibenförmige Ausbildung der Vorrichtung bedeutet im Sinne der vorliegenden Erfindung, dass die bestimmungsgemäß zusammengefügten Gehäuseteile in zwei orthogonal zueinander stehenden Richtungen jeweils eine größere Erstreckung aufweisen als in einer orthogonal zu den beiden Richtungen ausgebildeten dritten Richtung.

In weiterer Ausbildung der Erfindung weist die Vorrichtung wenigstens ein schnappendes, rastendes oder in sonstiger Weise hintergreifend wirkendes Verriegelungsmittel auf, mittels dessen der zweite Gehäuseteil gegen den ersten Gehäuseteil lösbar fixierbar ist. Hierdurch werden die beiden Gehäuseteile in ihrem bestimmungsgemäß zusammengefügten Zustand gehalten. Zum Lösen der zusammengefügten Gehäuseteile ist das Verriegelungsmittel freigebend betätigbar.

Vorzugsweise umfasst das wenigstens eine Verriegelungsmittel eine rückfedernd auslenkbare Verriegelungslasche und ein dazu komplementäres hintergreifbares Element. Die Verriegelungslasche kann insbesondere derart ausgebildet sein, dass sie beim translatorischen Aufschieben des einen, insbesondere zweiten Gehäuseteils auf den anderen, insbesondere ersten Gehäuseteil vorzugsweise selbsttätig ausgelenkt wird und anschließend dann in eine die Gehäuseteile gegeneinander verriegelnde Stellung einrastet.

In weitergehender Ausbildung der Erfindung erweist es sich als vorteilhaft, dass in einem Endbereich von ersten und zweiten Schiebeführungsmittel des ersten und zweiten Gehäuseteils zusammenwirkende Verriegelungsmittel ausgebildet sind. Die Verriegelungsmittel können insbesondere schnappende, rastende oder in sonstiger Weise hintergreifend wirkende Verriegelungsmittel sein.

Vorzugsweise bilden die Verriegelungsmittel einen Hintergriff quer zu einer translatorischen Schieberichtung. Auf diese Weise werden die im bestimmungsgemäß gefügten Zustand befindlichen Gehäuseteile verriegelt aneinander gehalten. Vorzugsweise ist das zweite Gehäuseteil auf diese Weise in Kombination mit dem weiteren Verriegelungsmittel, insbesondere der Verriegelungslasche, gesichert am ersten Gehäuseteil gehalten.

Vorzugsweise ist die Vorrichtung, insbesondere in einer mobilen Trageposition, am Körper eines Benutzers tragbar und mitführbar. Wenn vorstehend von einer tragbaren und mitführbaren Vorrichtung die Rede ist, so bedeutet dies, dass der Patient die Vorrichtung in einer Trageposition am Körper mitführen kann, so dass er mobil ist und dennoch seine Wunde dauerhaft, d.h. ohne Unterbrechung, therapiert werden kann. Die tragbare Vorrichtung der hier in Rede stehenden Art kann aber auch im stationären Betrieb, also losgelöst vom Körper des Patienten eingesetzt werden; sie kann solchenfalls beispielsweise an einem Pflegebett befestigt oder neben dem Pflegebett abgestellt werden. Unter einer Trageposition wird eine Trageposition der Vorrichtung am Körper eines aufrecht stehenden Benutzers verstanden, wobei die Vorrichtung insbesondere taschenartig wie eine Tasche über die Schulter des Benutzers gehängt getragen werden kann. Dies bedeutet aber nicht, dass die Vorrichtung nicht auch im Sitzen oder Liegen am Körper des Benutzers gehalten sein kann. Im Sinne der vorliegenden Erfindung erweist es sich als vorteilhaft, dass die bestimmungsgemäß zusammengefügten Gehäuseteile in der Tragesituation oder Trageposition am Körper eines Benutzers in der Weise orientiert sind, dass sie jeweils eine Breite und Höhe aufweisen, die gegenüber einer Tiefe in horizontaler Richtung betrachtet größer sind.

In weitergehender Ausbildung der Erfindung erweist es sich als vorteilhaft, dass der erste und der zweite Gehäuseteil der Vorrichtung im bestimmungsgemäß gefügten Zustand in der mobilen Trageposition betrachtet vertikal übereinander angeordnet sind, so dass zwischen den Gehäuseteilen eine im Wesentlichen horizontale Trennebene ausgebildet ist, wobei ein oberer Teil der Vorrichtung von dem ersten, die Unterdruck erzeugende Einrichtung umfassenden Gehäuseteil und ein unterer Teil der Vorrichtung von dem zweiten den Behälter bildenden Gehäuseteil gebildet wird. Wenn vorausgehend von einer Trennebene die Rede ist, so bedeutet dies aber nicht zwangsläufig, dass die Gehäuseteile über eine exakt ebene Fläche gegeneinander anliegen. Vielmehr können im Bereich der Trennebene Anschlüsse zur Unterdruckkommunikation oder sonstige Passungen, insbesondere der Schenkel bzw. die Schenkelplatte des L-förmigen Gehäuseteils als Schiebesitzführung, zwischen den beiden Gehäuseteilen ausgebildet sein. Die Gehäuseteile liegen im bestimmungsgemäß gefügten Zustand über die genannte im Wesentlichen horizontal orientierte Trennebene übereinander und gegeneinander an, wenn die Vorrichtung in der Trageposition am Körper eines aufrecht stehenden Menschen getragen wird. Auch wenn einer der Gehäuseteile L-förmig ausgebildet ist, so befindet sich der die Unterdruck erzeugende Einrichtung umfassende Bereich des ersten Gehäuseteils oberhalb des Behälters zur Aufnahme von Flüssigkeiten.

Nach einem weiteren Erfindungsgedanken von wesentlicher Bedeutung umfasst die Vorrichtung eine in einer mobilen Trageposition dem Körper des Benutzers zugewandte Rückseite und eine dieser gegenüberliegende, vom Körper des Benutzers abgewandte, eine Sichtseite der Vorrichtung bildende Vorderseite, wobei die Sichtseite der Vorrichtung zumindest teilweise durch einen Schenkel, insbesondere eine Schenkelplatte, des L-förmigen Gehäuseteils und die Rückseite zumindest teilweise durch eine hintere Wand des anderen Gehäuseteils gebildet wird.

Weiter erweist es sich als vorteilhaft, dass in mindestens einer Wandung des zweiten Gehäuseteils mindestens ein transparenter Bereich ausgebildet ist, durch den eine Einsichtnahme in das Innere des Behälters dann möglich ist, wenn dieser transparente Bereich nicht verdeckt ist. Eine Einsehbarkeit in das Innere des Behälters ermöglicht dem Benutzer eine schnelle und unkomplizierte Beurteilung der im Behälter vorhandenen Flüssigkeiten. Die Wandung des zweiten Gehäuseteils umfasst dafür einen hinreichend durchsichtigen Bereich, insbesondere ein Sichtfenster, durch den eine Einsichtnahme möglich ist, wenn dieser transparente Bereich nicht verdeckt ist.

Es erweist sich als besonders vorteilhaft, dass die Sichtseite und/oder die Rückseite der Vorrichtung aus einem nicht transparenten Material gefertigt ist, und im bestimmungsgemäß zusammengefügten Zustand die Schenkelplatte des ersten Gehäuseteils den zweiten Gehäuseteil in frontaler Blickrichtung auf die körperabgewandte Sichtseite der Vorrichtung zu wenigstens 90% oder vorzugsweise vollständig verdeckt. Hierdurch kann die gesamte Sichtseite der Vorrichtung im Wesentlichen von einer durchgehenden Oberfläche, insbesondere von der Schenkelplatte, gebildet werden, so dass insbesondere keine Schmutz aufnehmenden Fugen entstehen und ein einheitliches Erscheinungsbild resultiert. Ferner ist ein Einblick in das Innere des den Behälter bildenden zweiten Gehäuseteils für Dritte weitestgehend unmöglich, wenn der zweite Gehäuseteil in der Trageposition oder Tragesituation körperzugewandt orientiert ist und in frontaler Blickrichtung auf die körperabgewandte Sichtseite vorzugsweise vollständig von der Schenkelplatte des ersten Gehäuseteils in dieser Blickrichtung verdeckt ist.

Nach einem weiteren Erfindungsgedanken wird vorgeschlagen, dass die Vorrichtung ein gurtartiges Haltemittel aufweist, so dass sie am Körper des Benutzers tragbar und mitführbar ist. Das gurtartige Haltemittel ist vorzugsweise ein flexibles streifenförmiges, und vorzugsweise längenverstellbares Haltemittel, insbesondere aus Nylongewebe oder aus einem anderen an sich beliebigen geeigneten biegsamen Material. Unter einem flexiblen Haltemittel ist dabei ein Haltemittel zu verstehen, welches nicht formstabil sondern biegsam ist, welches sich also beim Tragen durch einen Patienten dessen Körperform anzupassen vermag. Ferner ist bevorzugt, wenn das Haltemittel eine, insbesondere gepolsterte, Schulterauflage umfasst, um den Tragekomfort der Vorrichtung zu verbessern. Vorzugsweise erhält die Vorrichtung durch das gurtartige Haltemittel in Kombination mit der Scheibenform der bestimmungsgemäß zusammengefügten Gehäuseteile ein taschenartiges Erscheinungsbild.

Nach einem weiteren Erfindungsgedanken von besonders großer Bedeutung erweist es sich als vorteilhaft, dass das Haltemittel derart an der Vorrichtung lösbar fixierbar ist, dass das Haltemittel in einer Betriebsstellung den transparenten Bereich einer Wandung des zweiten Gehäuseteils abdeckt, durch den eine Einsichtnahme in das Innere des Behälters möglich ist, wenn sich das Haltemittel nicht in der Betriebsstellung befindet. Ist das Haltemittel in der Betriebsstellung angeordnet, deckt es den transparenten Bereich in der Wandung des zweiten Gehäuseteils gewolltermaßen ab, um eine unerwünschte Einsichtnahme in das Innere des Behälters zu verhindern.

In weitergehender Ausbildung der Erfindung erweist es sich als vorteilhaft, dass das Haltemittel in der Betriebsstellung eine zwischen der Rückseite und der Vorderseite der Vorrichtung angeordnete Umfangsseite zumindest teilweise umläuft, so dass die Umfangsseite der Vorrichtung zumindest teilweise von dem Haltemittel bedeckt ist. Vorzugsweise umläuft das Haltemittel die Vorrichtung derart, dass in der mobilen Trageposition betrachtet ein unten liegender Bereich der Umfangsseite und seitlich liegende Bereiche der Umfangsseite von dem Haltemittel zumindest teilweise, vorzugsweise vollständig, bedeckt werden.

Vorzugsweise ist der transparente Bereich der Wandung des zweiten Gehäuseteils in der Umfangsseite der Vorrichtung ausgebildet. Die Umfangsseite der Vorrichtung verbindet die in der mobilen Trageposition dem Körper des Benutzers zugewandte Rückseite mit der dieser gegenüberliegenden vom Körper des Benutzers abgewandten und eine Sichtseite der Vorrichtung bildenden Vorderseite. Der transparente Bereich kann an beliebiger Stelle in der Umfangsseite ausgebildet sein. Vorzugsweise ist der transparente Bereich in einem in der mobilen Trageposition seitlich und/oder unten liegenden Bereich der Umfangsseite ausgebildet. Ist der zweite Gehäuseteil in der Trageposition oder Tragesituation körperzugewandt orientiert und in frontaler Blickrichtung auf die körperabgewandte Sichtseite vorzugsweise vollständig von der Schenkelplatte des ersten Gehäuseteils in dieser Blickrichtung verdeckt, kann in Kombination mit dem gurtartigen Haltemittel, welches vorzugsweise die Umfangsseite der Vorrichtung umläuft, eine unmittelbare Einsichtnahme in das Innere des Behälters auch dann verhindert werden, wenn der zweite Gehäuseteil oder der Behälter an mehreren Seiten oder insbesondere ganz durchsichtig ausgebildet ist.

Vorzugsweise weist die Vorrichtung Führungselemente für das Haltemittel auf. Die Führungselemente können insbesondere von abgewinkelten Stegen und/oder Laschen gebildet sein.

In weitergehender Ausbildung der Erfindung erweist es sich als vorteilhaft, dass ein Führungselement entlang der Umfangsseite in Umfangsrichtung erstreckt ist. Hierdurch ist es möglich, dass das Haltemittel die Vorrichtung in Umfangsrichtung geführt umläuft.

Vorzugsweise ist das Führungselement durch eine über die Umfangsseite vorspringende die Vorder- und/oder Rückseite bildende Deckplatte ausgebildet. Die Umfangsseite der Vorrichtung ist gegenüber der Deckplatte dann zurückgesetzt, so dass auf diese Weise ein entlang der Umfangsseite der Vorrichtung schienenartig erstrecktes Führungselement ausgebildet ist.

In weitergehender Ausbildung der Erfindung erweist es sich als vorteilhaft, dass die Vorrichtung Befestigungselemente, insbesondere Haken-/Schlaufen-Befestigungselemente, aufweist, mittels derer das Haltemittel lösbar an der Vorrichtung fixierbar ist. Um Einblick in das Innere des Behälters zu gewähren, kann die Fixierung des Haltemittels gelöst werden.

Vorzugsweise ist auf der Rückseite der Vorrichtung eine Ausnehmung für die zum Körper führende Saugleitung ausgebildet.

Vorzugsweise weist die Vorrichtung, insbesondere in einem in der Trageposition oberen Teil der Umfangsseite, welcher von dem in der Betriebsstellung befindlichen Haltemittel nicht überdeckt wird, Bedien- und Anzeigeelemente auf. Hierdurch sind auch der Zugriff und die Bedienung der Vorrichtung durch den Benutzer der Vorrichtung selbst möglich, indem die Bedienkomponenten und Anzeigekomponenten dann körperabgewandt und vorzugsweise von oben einsehbar angeordnet sind.

Neben der Unterdruckwundbehandlung sind auch andere Anwendungen der hier in Rede stehenden Vorrichtung zur Bereitstellung von Unterdruck für medizinische Anwendungen denkbar, insbesondere die Absaugung an sich beliebiger Körperflüssigkeiten, im Bereich der medizinischen Versorgung, insbesondere Inkontinentenversorgung, der Versorgung von Stoma-Patienten oder im Bereich der Absaugung von Wundsekreten, gegebenenfalls unter Verwendung von Spülflüssigkeiten, auch ohne Anlegen eines Unterdrucks über wesentliche Zeiträume.

Weitere Vorteile der Erfindung ergeben sich aus den beigefügten Patentansprüchen und aus der zeichnerischen Darstellung und nachfolgenden Beschreibung einer bevorzugten Ausführungsform der erfindungsgemäßen Vorrichtung. In der Zeichnung zeigt:
- Fig. 1a bis 1c: verschiedene Ansichten einer bevorzugten Ausführungsform der erfindungsgemäßen am Körper eines Benutzers tragbaren Vorrichtung zur Bereitstellung von Unterdruck für medizinische Anwendungen, und
- Fig. 2a bis 2g: verschiedene Ansichten eines eine Unterdruck erzeugende Einrichtung und Steuerungskomponenten umfassenden ersten Gehäuseteils und eines einen Behälter zur Aufnahme von Körperflüssigkeiten bildenden zweiten Gehäuseteils der Vorrichtung nach Figuren 1a bis 1c.

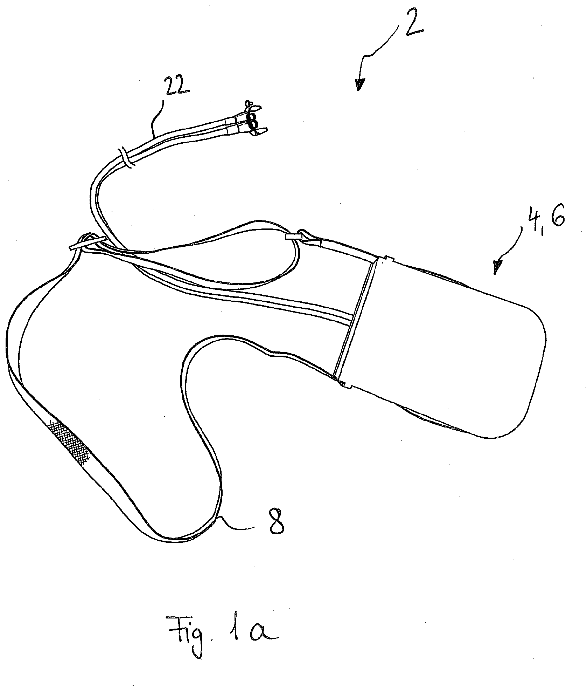

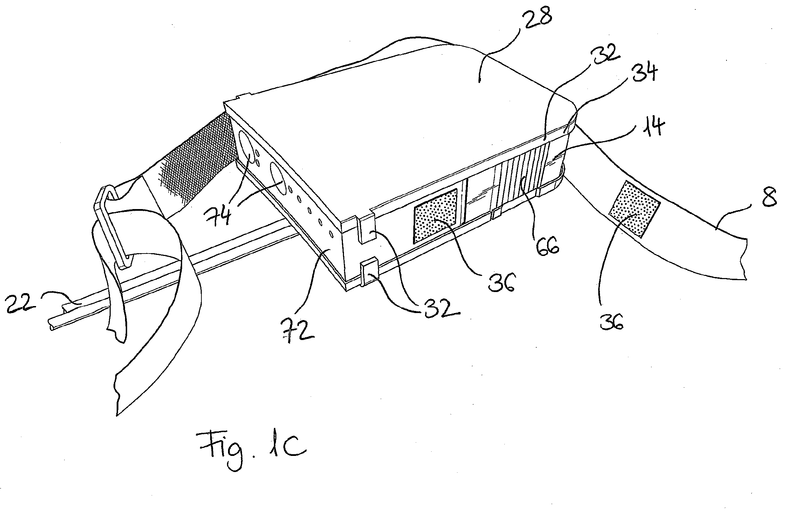

Die Figuren 1a bis c zeigen eine erste Ausführungsform einer erfindungsgemäßen tragbaren Vorrichtung 2 zur Bereitstellung von Unterdruck für medizinische Anwendungen. Die Vorrichtung 2 umfasst zwei lösbar gegeneinander fixierbare Gehäuseteile 4, 6, sowie ein gurtartiges Haltemittel 8, so dass die Vorrichtung 2 am Körper eines Benutzers tragbar und mitführbar ist. Das Haltemittel 8 ist vorzugsweise ein flexibles streifenförmiges, riemenartiges längenverstellbares Haltemittel 8, insbesondere aus Nylongewebe oder einem beliebigen anderen Material. Ferner ist es bevorzugt, wenn das Haltemittel eine, insbesondere gepolsterte, Schulterauflage (nicht dargestellt) umfasst. Hierdurch wird der Tragekomfort der Vorrichtung weiter verbessert.

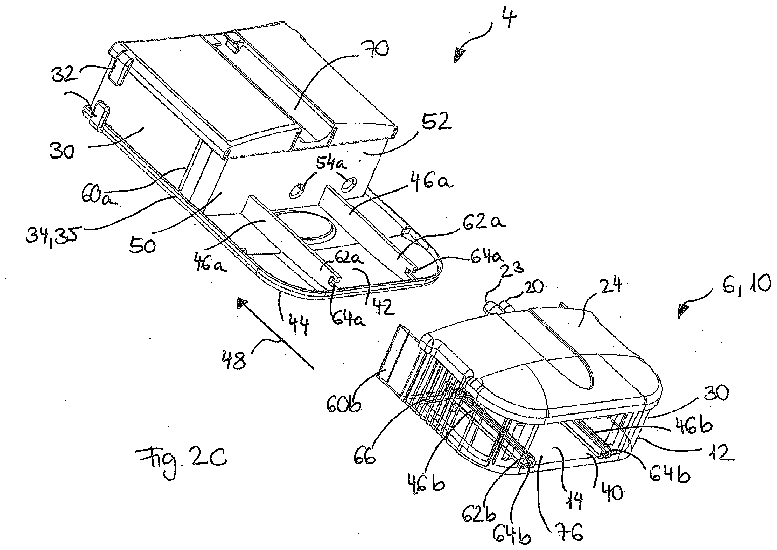

Fig. 2c zeigt die Gehäuseteile 4, 6 in einer separat voneinander angeordneten Position. In dem ersten Gehäuseteil 4 ist eine Unterdruck erzeugende Einrichtung vorzugsweise in Form einer Luftpumpe sowie elektrische und elektronische Steuerkomponenten für die Vorrichtung 2 einschließlich Batterien oder vorzugsweise wiederaufladbarer Akkus aufgenommen. Der zweite Gehäuseteil 6 bildet im bevorzugten Fall zugleich einen Behälter 10 zur Aufnahme von Körperflüssigkeiten, insbesondere zur Aufnahme von aus einer Wunde abgesaugten Wundsekreten.

In einer Wandung 12 des zweiten Gehäuseteils 6 ist ein transparenter Bereich 14 ausgebildet, durch den eine Einsichtnahme in das Innere 16 des Behälters 10 möglich ist, um insbesondere den Füllstand im Behälter 10 zu überprüfen. Vorzugsweise ist der gesamte zweite Gehäuseteil 6 als wegwerfbarer Einwegartikel ausgebildet. An einem oberen Bereich 18 des zweiten Gehäuseteils 6 ist ein Anschlussstutzen 20 für eine Saugleitung 22 (vgl. Fig. 1a) vorgesehen, die dann beispielsweise bei der Verwendung der Vorrichtung 2 zur Unterdrucktherapie von Wunden zu einem die Wunde druckdicht verschließenden Wundverband führt und dort beispielsweise über einen Port mit dem Wundraum kommuniziert, um im Wundraum einen Unterdruck anzulegen und aufrechtzuerhalten und Wundsekrete in den Behälter 10 abzusaugen. Hierfür kommuniziert der Behälter 10 mit der Unterdruck erzeugenden Einrichtung, die in dem ersten Gehäuseteil 4 angeordnet ist. Bei dem weiteren Anschlussmittel 23 kann es sich um einen Instillations- oder Spülanschluss oder um einen Druckmessanschluss handeln.

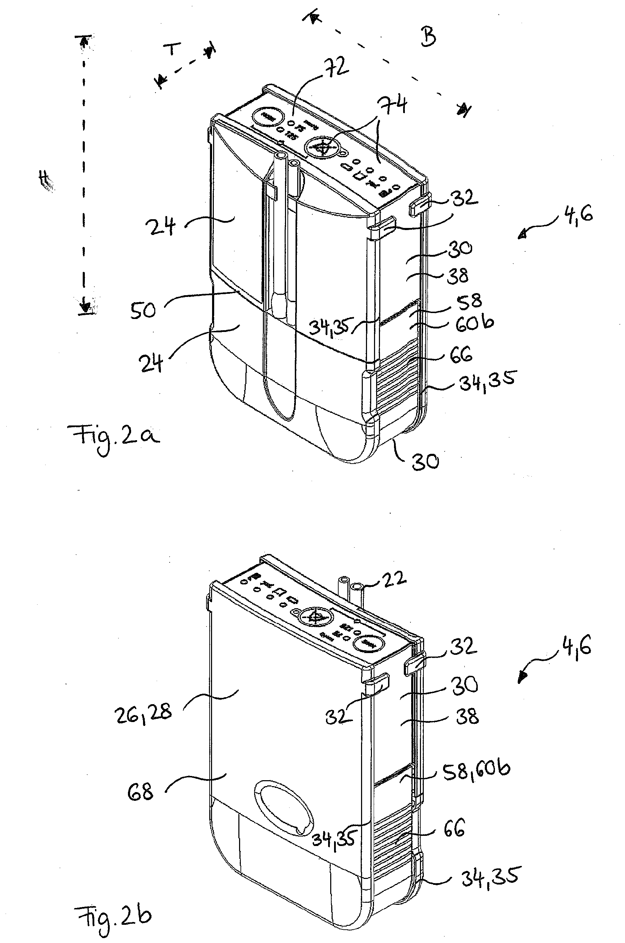

In der in den Fig. 1a bis 1c und 2a, 2b gezeigten Ausführungsform befinden sich die Gehäuseteile 4, 6 in einem bestimmungsgemäß zusammengefügten Zustand. In einer mobilen Trageposition oder Tragesituation, in der die Vorrichtung 2 beispielsweise wie eine Tragetasche über eine Schulter des Benutzers gehängt getragen wird, weist die Vorrichtung 2 eine dem Körper des Benutzers zugewandte Rückseite 24 und eine dieser gegenüberliegende vom Körper des Benutzers abgewandte und eine Sichtseite 26 der Vorrichtung bildende Vorderseite 28 auf. Zwischen der Vorder- und Rückseite 28, 24 ist eine diese verbindende Umfangsseite 30 angeordnet. Vorzugsweise ist der transparente Bereich 14 der Wandung 12 des zweiten Gehäuseteils 6 in der Umfangsseite 30 der Vorrichtung 2 angeordnet. Das gurtartige Haltemittel 8 der Vorrichtung 2 ist in einer Betriebsstellung erfindungsgemäß derart angeordnet, dass es den transparenten Bereich 14 des zweiten Gehäuseteils 6 überdeckt und eine Einsichtnahme in das Innere 16 des Behälters 10 daher verhindert ist. Gemäß der Fig. 1a umgibt das Haltemittel 8 in der Betriebsstellung die Vorrichtung 2 im Bereich der Umfangsseite 30 umfänglich zumindest teilweise, so dass die Umfangsseite 30 der Vorrichtung 2 teilweise und der transparente Bereich 14 vollständig von dem Haltemittel 8 bedeckt ist.

Des Weiteren zeigen die Figuren 1a bis 2e im Bereich der Umfangsseite 30 Führungselemente 32 für das gurtartige Haltemittel 8, insbesondere und vorzugsweise in Form von abgewinkelten Stegen und/oder Laschen, hinter welche das Haltemittel 8 gebracht und somit geführt zwischen diesen und der Umfangsseite 30 angeordnet werden kann. Es erweist sich als vorteilhaft, wenn die Umfangsseite 30 in Bezug auf einen äußeren Rand 34 einer die Vorder- oder Rückseite 28, 24 bildende Deckplatte der Vorrichtung 2 zurückversetzt angeordnet ist, so dass auf diese Weise ein Führungselement 32 in Form einer in Umfangsrichtung erstreckten Schiene oder Rippe 35 gebildet ist, welche das Haltemittel 8 entlang der Umfangsrichtung insbesondere beidseits begrenzt und führt und in der gewünschten Betriebsstellung hält.

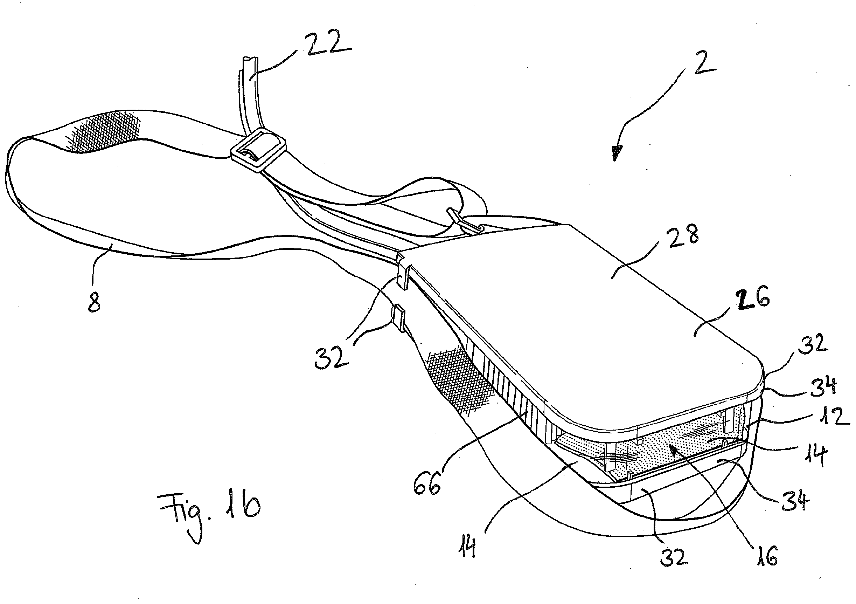

Ferner umfasst die Vorrichtung 2 Befestigungselemente 36, insbesondere Haken-/Schlaufen-Befestigungselemente oder Druckknöpfe, mit welchen das Haltemittel 8 an der Vorrichtung 2 lösbar fixierbar ist und in der Betriebsstellung gehalten werden kann, so dass das Haltemittel 8 nicht unbeabsichtigt verrutschen und einen unerwünschten Blick in das Innere 16 des Behälters 10 freigeben kann. Um eine gewollte Einsichtnahme in den Behälter 10 zu ermöglichen, kann das Haltemittel 8 von den Befestigungselementen 36 gelöst und aus der Betriebsstellung bewegt werden. Es erweist sich als vorteilhaft, wenn das Haltemittel 8 nicht komplett von den Gehäuseteilen 4, 6 abgenommen werden muss, um einen Blick in das Innere 16 des Behälters 10 freizugeben. Vorzugsweise umfasst die Vorrichtung 2 mehrere Befestigungselemente 36, die im Bereich der Umfangsseite 30, insbesondere an seitlichen Bereichen 38 und/oder an einem unteren Bereich 40 der Umfangsseite 30, angeordnet sind. In einer bevorzugten Ausführungsform kann das Haltemittel 8 an mindestens einem der Befestigungselemente 36 fixiert und um die Umfangsseite 30 herum angeordnet bleiben und nur teilweise aus der Betriebsstellung bewegt werden, um eine Einsichtnahme in den Behälter 10 zu ermöglichen (Fig. 1b).

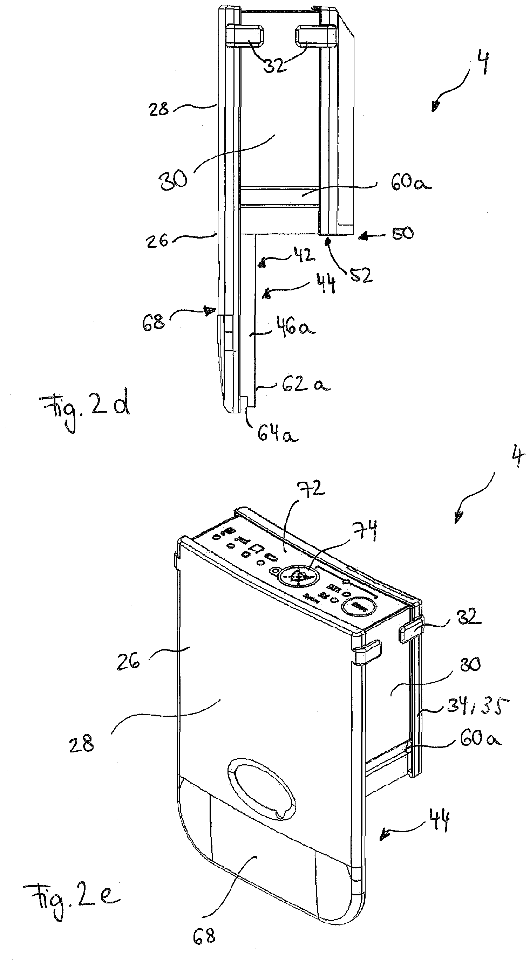

Man erkennt des Weiteren bei der dargestellten bevorzugten Ausführungsform in den Figuren 2c, 2d und 2e, dass der erste Gehäuseteil 4 in der Seitenansicht ein L-förmiges Profil mit einer Schenkelplatte 44 aufweist, deren Breite und Höhe gegenüber der Tiefe in horizontaler Richtung in der Trageposition betrachtet größer sind. Auf einer Innenseite 42 der Schenkelplatte 44 des L-förmigen ersten Gehäuseteils 4 sind beispielhaft zwei erste Schiebeführungsmittel 46a ausgebildet. Der zweite Gehäuseteil 6 umfasst zu den ersten Schiebeführungsmitteln 46a komplementäre zweite Schiebeführungsmittel 46b. Hierdurch ist der zweite Gehäuseteil 6 auf die Schenkelplatte 44 des L-förmigen ersten Gehäuseteils 4 translatorisch geführt in einer Schieberichtung 48 aufschiebbar. Die ersten Schiebeführungsmittel 46a des ersten Gehäuseteils 4 sind in der gezeigten Ausführungsform als Führungsschienen oder -rippen ausgebildet. Die zweiten Schiebeführungsmittel 46b sind als dazu komplementäre Aufnahmevertiefungen, insbesondere in Form von Schiebeführungsnuten, ausgebildet. Wenn die ersten Schiebeführungsmittel 46a in Eingriff mit den zweiten Schiebeführungsmitteln 46b sind, lässt sich der zweite Gehäuseteil 6 translatorisch auf den ersten Gehäuseteil 4 aufschieben, bis die Gehäuseteile im bestimmungsgemäß zusammengesetzten Zustand (Fig. 2a und 2b) auf Stoß aneinander anliegen und der zweite Gehäuseteil 6 das L-förmige erste Gehäuseteil 4 komplementär zu einer Art Scheibenform ergänzt. Scheibenform im Sinne der vorliegenden Erfindung bedeutet, dass die Breite B der Vorrichtung 2 in der Trageposition betrachtet in horizontaler Richtung und die Höhe H in vertikaler Richtung der zusammengefügten Gehäuseteile jeweils größer ist als die Tiefe T in horizontaler Richtung und orthogonal zur Breiten- und Höhenerstreckung. Hierdurch ist es möglich, dass die Vorrichtung 2 in Tiefenrichtung insgesamt so ausgebildet und bemessen werden kann, dass sie bequem und taschenartig am Körper eines Benutzers getragen werden kann.

Betrachtet man die Vorrichtung 2 in der Trageposition am Körper des Benutzers, so liegen die Gehäuseteile 4, 6 über eine im Wesentlichen horizontale Trennebene 50 gegeneinander an, wobei sich die Schenkelplatte 44 des ersten Gehäuseteils 4 plattenartig vorspringend über eine Erstreckung des ersten Gehäuseteils 4 hinaus fortsetzt, um ein Führungsmittel zum Fügen der beiden Gehäuseteile zu bilden.
Dessen ungeachtet befindet sich aber der die Unterdruck erzeugende Einrichtung umfassende Bereich des ersten Gehäuseteils 4 oberhalb der Trennebene 50 und der zweite Gehäuseteil 6 unterhalb der Trennebene 50.

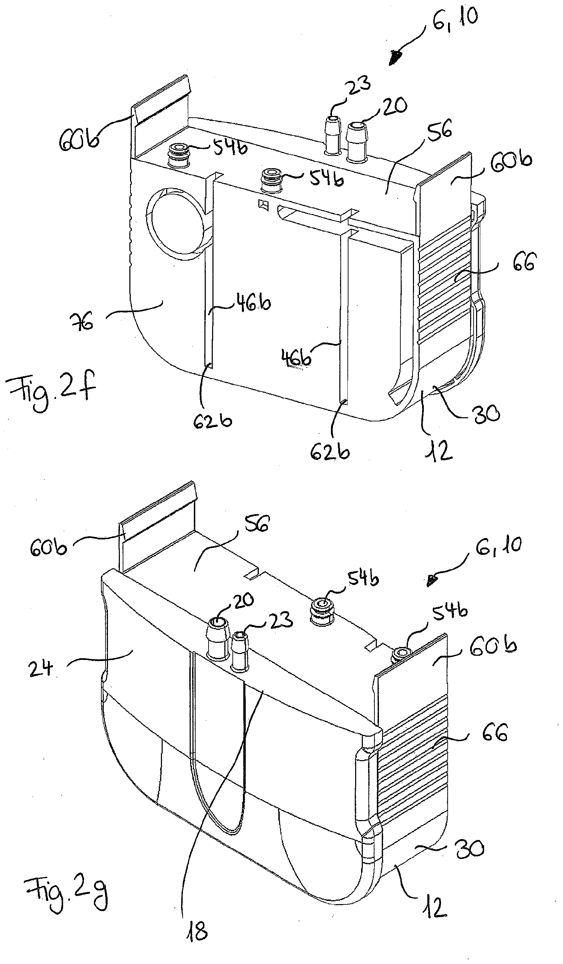

Aus der Figur 2c, welche den ersten Gehäuseteil 4 separat von dem zweiten Gehäuseteil 6 zeigt, ist unmittelbar ersichtlich, dass auf einer dem zweiten Gehäuseteil 6 zugewandten Seite 52 des ersten Gehäuseteils 4 mehrere Anschlussmittel 54a ausgebildet sind, die mit Anschlussmitteln 54b (Fig. 2f) des zweiten Gehäuseteils koppelbar sind, wenn die Gehäuseteile miteinander gefügt werden. Die dem ersten Gehäuseteil 4 zugewandte Seite 56 des zweiten Gehäuseteils 6 ist komplementär zu der Ausbildung der Seite 52 des ersten Gehäuseteils 4 ausgebildet, so dass die beiden Gehäuseteile 4, 6 nur in einer korrekten Weise miteinander gefügt bzw. aneinander befestigt werden können.

Des Weiteren umfasst die Vorrichtung 2 schnappende, rastende oder in sonstiger Weise hintergreifend wirkende Verriegelungsmittel 58, mittels derer der zweite Gehäuseteil 6 gegen den ersten Gehäuseteil 4 lösbar fixierbar ist. Durch translatorisches Aufschieben des zweiten Gehäuseteils 6 auf den ersten Gehäuseteil 4, insbesondere im Wesentlichen quer zu der horizontalen Trennebene 50, werden am zweiten Gehäuseteil 6 angeordnete Verriegelungslaschen 60b, insbesondere selbsttätig, ausgelenkt und rasten dann in eine die Gehäuseteile 4, 6 gegeneinander verriegelnde Stellung. Hierfür sind an dem ersten Gehäuseteil 4 zu den Verriegelungslaschen 60b komplementäre hintergreifbare Elemente 60a vorgesehen, die von den Verriegelungslaschen 60b hintergriffen werden. Wenn die Gehäuseteile 4, 6 in ihre verriegelte Stellung gebracht sind, wird vorzugsweise automatisch eine Unterdruckkommunikation zwischen dem Inneren des Behälters 10 des zweiten Gehäuseteils 6 und der Unterdruck erzeugenden Einrichtung hergestellt.

Gemäß der bevorzugten gezeigten Ausführungsform sind in Endbereichen 62a, 62b der ersten und zweiten Schiebeführungsmittel 46a, 46b des ersten und zweiten Gehäuseteils 4, 6 weitere Verriegelungsmittel 64a, 64b ausgebildet. Die Endbereiche 62a, 62b der Schiebeführungsmittel 46a, 46b sind derart korrespondierend profiliert ausgebildet, dass diese ineinander eingreifen, wenn die beiden Gehäuseteile 4, 6 auf Stoß aneinander anliegen.

Die Figuren 2a und 2b zeigen die Gehäuseteile 4, 6 im bestimmungsgemäß zusammengefügten Zustand, in einer Schrägansicht auf die Vorderseite 28 bzw. Sichtseite 26 der Vorrichtung 2 (Fig. 2b) und in einer Schrägansicht auf die Rückseite 24 der Vorrichtung 2 (Fig. 2a). Im gefügten Zustand der beiden Gehäuseteile 4 und 6 befinden sich die Verriegelungsmittel 58, 60a, 60b, 64a und 64b in einem die beiden Gehäuseteile 4, 6 formschlüssig aneinander haltenden verriegelten Zustand. Es ist denkbar, dass Betätigungsorgane, beispielsweise Taster, vorgesehen sind, welche betätigbar sind, um die Verriegelungsmittel 58, 60a, 60b, 64a und 64b zu lösen. Vorzugsweise sind die Verriegelungsmittel 58, 60a, 60b, 64a und 64b selbsttätig lösbar, wenn der zweite Gehäuseteil 6 in einer translatorischen Zugbewegung in einer Richtung entgegen der Schieberichtung 48 von dem ersten Gehäuseteil 4 weggezogen wird.

Wie aus den Figuren 2a bis 2c, sowie 2f und 2g ersichtlich sind am zweiten Gehäuseteil 6 zwei Griffflächen 66 ausgebildet. Zum Lösen des zweiten Gehäuseteils 6 vom ersten Gehäuseteil 4, kann der Benutzer das zweite Gehäuseteil beidseitig an den Griffflächen 66 greifen und den zweiten Gehäuseteil 6 durch die translatorische Zugbewegung vom ersten Gehäuseteil 4 abziehen. Indem der zweite Gehäuseteil auf der einen Seite mit Daumen und auf der anderen Seite mit den restlichen Fingern im Bereich der Griffflächen 66 gegriffen wird, kann der zweite Gehäuseteil 6 mit einer Hand vom ersten Gehäuseteils 4 abgenommen werden. Dies erweist sich als besonders vorteilhaft, da auf diese Weise ein mit Körperflüssigkeiten befüllter Behälter 10 mit nur einer Hand gelöst und in ein Entsorgungsbehältnis gegeben werden kann.

Gleichwohl sind die Gehäuseteile 4, 6 und die Verriegelungsmittel 58, 60a, 60b, 64a und 64b derart klemmschlüssig gegeneinander gehalten, dass ein unbeabsichtigtes Separieren der beiden Gehäuseteile 4, 6 voneinander verhindert ist. Ist das gurtartige Haltemittel 8 in der Betriebsposition angeordnet, so umläuft es (in den Fig. 2a bis 2g nicht dargestellt) die Umfangsseite 30 der Vorrichtung zumindest teilweise und verdeckt dabei den transparenten Bereich 14 in der Wandung 12 des zweiten Gehäuseteils 6. Das Haltemittel 8 ist über die Führungs- und/oder Befestigungselemente 32, 36 zumindest an dem ersten Gehäuseteil 4 fixiert.

In der bevorzugten gezeigten Ausführungsform wird die Sichtseite 26 der Vorrichtung 2 durch eine Außenseite 68 der Schenkelplatte 44 des L-förmigen ersten Gehäuseteils 4 gebildet, die vorzugsweise aus einem nicht transparenten Material gefertigt ist. In der mobilen Trageposition am Körper eines Benutzers ist somit ein Einblick in das Innere 16 des den Behälter 10 bildenden zweiten Gehäuseteils 6 für Dritte weitestgehend unmöglich, wenn der zweite Gehäuseteil 6 in frontaler Blickrichtung auf die körperabgewandte Sichtseite 26 vollständig von der Schenkelplatte 44 des ersten Gehäuseteils 4 in dieser Blickrichtung verdeckt ist und sich das gurtartige Haltemittel 8 in der Betriebsposition befindet. Eine Einsichtnahme ist selbst dann nicht möglich, wenn der zweite Gehäuseteil 6 zusätzlich zu dem transparenten Bereich 14 der Wandung 12 in der Umfangsseite 30 der Vorrichtung 2 weitere transparente Bereiche 76 (vgl. Fig. 2c) aufweist. Die transparenten Bereiche 14, 76 sind nur in den Figuren 2c und 1b als solche zeichnerisch dargestellt. Beispielsweise könnte die komplette Seite des zweiten Gehäuseteils, welche die zweiten Schiebeführungsmittel 46b umfasst, oder auch alle Seiten als transparenter Bereich 76 ausgebildet sein (vgl. Fig. 2f). Die Rückseite 24 der Vorrichtung 2 wird zum Teil durch den ersten und zum Teil durch den zweiten Gehäuseteil 4, 6 gebildet und ist vorzugsweise aus einem nicht transparenten Material gefertigt.

Der erste Gehäuseteil 4 weist eine langgestreckte kanalbildende Ausnehmung 70 für die am Anschlussstutzen 20 des zweiten Gehäuseteils 6 befestigte und zum Körper führende Saugleitung 22 und für die optionale Mess- und/oder Spülleitung auf.

In einem in der Trageposition betrachtet oben liegenden Bereich 72 der Umfangsseite 30, welcher von dem in der Betriebsstellung befindlichen Haltemittel 8 nicht überdeckt wird, sind Bedien- und Anzeigeelemente 74 vorgesehen, die für den Benutzer gut zugänglich und einsehbar sind, wenn sich die Vorrichtung 2 in der mobilen Trageposition am Körper eines Benutzers befindet.

## Patentansprüche

1. Vorrichtung (2) zur Bereitstellung von Unterdruck zur Unterdruckbehandlung von Wunden am menschlichen Körper, mit einer Unterdruck erzeugenden Einrichtung und einem Behälter (10) zur Aufnahme von Flüssigkeiten in einem Inneren (16) des Behälters (10), insbesondere von aus einer Wunde abgesaugten Wundsekreten, mit einem Anschluss (20) für eine zum Körper führende Saugleitung (22), wobei die Unterdruck erzeugende Einrichtung in einem ersten Gehäuseteil (4) der Vorrichtung (2) angeordnet ist und der Behälter (10) von einem zweiten Gehäuseteil (6) der Vorrichtung (2) gebildet ist, wobei der zweite Gehäuseteil (6) der Vorrichtung (2) an dem ersten Gehäuseteil (4) befestigbar und manuell lösbar ist und im befestigten Zustand das Innere (16) des Behälters (10) über einen Anschluss (54b) von der Unterdruck erzeugenden Einrichtung mit Unterdruck beaufschlagbar ist, so dass eine Unterdruckkommunikation zwischen der Unterdruck erzeugenden Einrichtung, dem Behälter (10) und der zum Körper führenden Saugleitung (22) herstellbar ist, wobei wenigstens ein Gehäuseteil (4, 6) in einer Seitenansicht im Wesentlichen L-förmig ausgebildet ist, **dadurch gekennzeichnet, dass** der L-förmige Gehäuseteil eine Schiebesitzführung für den anderen Gehäuseteil bildet, so dass die Gehäuseteile (4, 6) translatorisch in einen bestimmungsgemäß gefügten Zustand aneinander bringbar sind, und dass auf einer Innenseite (42) eines Schenkels (44) des L-förmigen Gehäuseteils wenigstens ein erstes Schiebeführungsmittel (46a) ausgebildet ist, und der andere Gehäuseteil ein zu dem ersten Schiebeführungsmittel (46a) komplementäres zweites Schiebeführungsmittel (46b) umfasst, so dass der andere Gehäuseteil auf den Schenkel (44) des L-förmigen Gehäuseteils translatorisch geführt aufschiebbar ist, wobei die Schiebeführungsmittel (46a, 46b) ineinander eingreifen, und dass das erste und das zweite Schiebeführungsmittel (46a, 46b) wenigstens eine Führungsschiene und wenigstens eine dazu komplementäre Führungsnut umfassen.

2. Vorrichtung (2) nach Anspruch 1, **dadurch gekennzeichnet, dass** der Schenkel (44) des L-förmigen Gehäuseteils durch eine Schenkelplatte gebildet ist, die insbesondere eine Dicke quer zu ihrer flächenhaften Erstreckung von höchstens 10mm, insbesondere höchstens 8mm, insbesondere höchstens 5mm, insbesondere höchstens 3mm, aufweist.

3. Vorrichtung (2) nach Anspruch 1 oder 2, **dadurch gekennzeichnet, dass** in dem bestimmungsgemäß gefügten Zustand der zweite Gehäuseteil (6) mit dem ersten Gehäuseteil (4) auf Stoß liegt.

4. Vorrichtung (2) nach einem oder mehreren der vorhergehenden Ansprüche, **dadurch gekennzeichnet, dass** der erste Gehäuseteil (4) in der Seitenansicht im Wesentlichen L-förmig ausgebildet ist und der zweite Gehäuseteil (6) ein den ersten Gehäuseteil (4) komplementär ergänzendes Profil aufweist, so dass die Vorrichtung (2) im Wesentlichen eine Scheibenform aufweist, wenn sich die Gehäuseteile (4, 6) in dem bestimmungsgemäß gefügten Zustand befinden.

5. Vorrichtung (2) nach einem oder mehreren der vorhergehenden Ansprüche, **dadurch gekennzeichnet, dass** die Vorrichtung (2) wenigstens ein schnappendes, rastendes oder in sonstiger Weise hintergreifend wirkendes Verriegelungsmittel (58) aufweist, mittels dessen der zweite Gehäuseteil (6) gegen den ersten Gehäuseteil (4) lösbar fixierbar ist.

6. Vorrichtung (2) nach Anspruch 5, **dadurch gekennzeichnet, dass** das wenigstens eine Verriegelungsmittel (58) eine rückfedernd auslenkbare Verriegelungslasche (60b) und ein dazu komplementäres hintergreifbares Element (60a) umfasst.

7. Vorrichtung (2) nach einem oder mehreren der vorhergehenden Ansprüche, **dadurch gekennzeichnet, dass** in einem Endbereich (62a, 62b) von ersten und zweiten Schiebeführungsmitteln (46a, 46b) des ersten und zweiten Gehäuseteils (4, 6) zusammenwirkende Verriegelungsmittel (64a, 64b) ausgebildet sind.

8. Vorrichtung (2) nach Anspruch 7, **dadurch gekennzeichnet, dass** die Verriegelungsmittel (64a, 64b) einen Hintergriff quer zu einer translatorischen Schieberichtung (48) bilden.

9. Vorrichtung (2) nach einem oder mehreren der vorhergehenden Ansprüche, **dadurch gekennzeichnet, dass** die Vorrichtung (2), insbesondere in einer mobilen Trageposition, am Körper eines Benutzers tragbar und mitführbar ist und dass der erste und der zweite Gehäuseteil (4, 6) der Vorrichtung (2) im bestimmungsgemäß gefügten Zustand in der mobilen Trageposition betrachtet vertikal übereinander angeordnet sind, so dass zwischen den Gehäuseteilen (4, 6) eine im Wesentlichen horizontale Trennebene (50) ausgebildet ist, wobei ein oberer Teil der Vorrichtung (2) von dem ersten, die Unterdruck erzeugende Einrichtung umfassenden Gehäuseteil (4) und ein unterer Teil der Vorrichtung von dem zweiten den Behälter (10) bildenden Gehäuseteil (6) gebildet wird.

10. Vorrichtung (2) nach einem oder mehreren der vorhergehenden Ansprüche, **dadurch gekennzeichnet, dass** die Vorrichtung (2) eine in einer mobilen Trageposition dem Körper des Benutzers zugewandte Rückseite (24) und eine dieser gegenüberliegende, vom Körper des Benutzers abgewandte, eine Sichtseite (26) der Vorrichtung (2) bildende Vorderseite (28) umfasst, wobei die Sichtseite (26) der Vorrichtung (2) zumindest teilweise durch einen Schenkel (44), insbesondere eine Schenkelplatte, des L-förmigen Gehäuseteils und die Rückseite (24) zumindest teilweise durch eine hintere Wand des anderen Gehäuseteils gebildet wird.

11. Vorrichtung (2) nach einem der vorhergehenden Ansprüche, **dadurch gekennzeichnet, dass** in mindestens einer Wandung (12) des zweiten Gehäuseteils (6) mindestens ein transparenter Bereich (14) ausgebildet ist, durch den eine Einsichtnahme in das Innere (16) des Behälters (10) dann möglich ist, wenn dieser transparente Bereich (14) nicht verdeckt ist.

12. Vorrichtung (2) nach Anspruch 10, **dadurch gekennzeichnet, dass** die Sichtseite (26) und/oder die Rückseite (24) der Vorrichtung (2) aus einem nicht transparenten Material gefertigt ist, und im bestimmungsgemäß zusammengefügten Zustand die Schenkelplatte (44) des ersten Gehäuseteils (4) den zweiten Gehäuseteil (6) in frontaler Blickrichtung auf die körperabgewandte Sichtseite (26) der Vorrichtung (2) zu wenigstens 90% oder vorzugsweise vollständig verdeckt.

13. Vorrichtung (2) nach einem oder mehreren der vorhergehenden Ansprüche, **dadurch gekennzeichnet, dass** die Vorrichtung (2) ein gurtartiges Haltemittel (8) aufweist, so dass sie am Körper des Benutzers tragbar und mitführbar ist.

14. Vorrichtung (2) nach Anspruch 13, **dadurch gekennzeichnet, dass** das Haltemittel (8) derart an der Vorrichtung (2) lösbar fixierbar ist, dass das Haltemittel (8) in einer Betriebsstellung einen transparenten Bereich (14) einer Wandung (12) des zweiten Gehäuseteils (6) abdeckt, durch den eine Einsichtnahme in das Innere (16) des Behälters (10) möglich ist, wenn sich das Haltemittel (8) nicht in der Betriebsstellung befindet.

15. Vorrichtung (2) nach Anspruch 13 oder 14, **dadurch gekennzeichnet, dass** das Haltemittel (8) in der Betriebsstellung eine zwischen der Rückseite (24) und der Vorderseite (28) der Vorrichtung (2) angeordnete Umfangsseite (30) zumindest teilweise umläuft, so dass die Umfangsseite (30) der Vorrichtung (2) zumindest teilweise von dem Haltemittel (8) bedeckt ist.

## Claims

1. Device (2) for providing a vacuum for vacuum-treating wounds on the human body, comprising a vacuum-generating apparatus and a container (10) for receiving liquids in an interior (16) of the container (10), in particular wound secretions suctioned from a wound, said container comprising a connection (20) for a suction line (22) leading to the body, the vacuum-generating apparatus being arranged in a first housing part (4) of the device (2) and the container (10) being formed by a second housing part (6) of the device (2), it being possible for the second housing part (6) of the device (2) to be fastened to the first housing part (4) and manually released and, in the fastened state, for a vacuum to be applied to the interior (16) of the container (10) via a connection (54b) by the vacuum-generating apparatus, such that a vacuum communication can be established between the vacuum-generating apparatus, the container (10) and the suction line (22) leading to the body, at least one housing part (4, 6) being substantially L-shaped in a side view, **characterized in that** the L-shaped housing part forms a sliding seat guide for the other housing part, such that the housing parts (4, 6) can be brought together in a translational manner into a state in which they are joined as intended, and **in that** at least one first sliding guide means (46a) is formed on an inner face (42) of a leg (44) of the L-shaped housing part, and the other housing part comprises a second sliding guide means (46a) which is complementary to the first sliding guide means (46b), such that the other housing part can be pushed onto the leg (44) of the L-shaped housing part in a translationally guided manner, the sliding guide means (46a, 46b) engaging in one another, and **in that** the first and the second sliding guide means (46a, 46b) comprise at least one guide rail and at least one guide groove which is complementary thereto.

2. Device (2) according to claim 1, **characterized in that** the leg (44) of the L-shaped housing part is formed by a leg plate which in particular has a thickness transverse to the planar extension thereof of at most 10 mm, in particular at most 8 mm, in particular at most 5 mm, in particular at most 3 mm.

3. Device (2) according to claim 1 or claim 2, **characterized in that** the second housing part (6) is in abutment with the first housing part (4) when they are joined as intended.

4. Device (2) according to one or more of the preceding claims, **characterized in that** the first housing part (4) is substantially L-shaped in the side view and the second housing part (6) has a profile which is added to the first housing part (4) in a complementary manner such that the device (2) is substantially disc-shaped when the housing parts (4, 6) are joined as intended.

5. Device (2) according to one or more of the preceding claims, **characterized in that** the device (2) has at least one locking means (58) which snaps, latches or engages in some other manner, by means of which the second housing part (6) can be releasably fixed against the first housing part (4).

6. Device (2) according to claim 5, **characterized in that** the at least one locking means (58) comprises a resiliently deflectable locking tab (60b) and an engageable element (60a) which is complementary thereto.

7. Device (2) according to one or more of the preceding claims, **characterized in that** interacting locking means (64a, 64b) are formed in an end region (62a, 62b) of the first and second sliding guide means (46a, 46b) of the first and second housing part (4, 6).

8. Device (2) according to claim 7, **characterized in that** the locking means (64a, 64b) form an engagement transversely to a translational sliding direction (48).

9. Device (2) according to one or more of the preceding claims, **characterized in that** the device (2), in particular in a mobile wearing position, can be worn on the body of the user and carried around, and **in that** the first and the second housing parts (4, 6) of the device (2), when joined as intended, are arranged vertically one above the other when viewed in the mobile wearing position, such that a substantially horizontal separating plane (50) is formed between the housing parts (4, 6), an upper part of the device (2) being formed by the first housing part (4) comprising the vacuum-generating apparatus and a lower part of the device being formed by the second housing part (6) which forms the container (10) .

10. Device (2) according to one or more of the preceding claims, **characterized in that** the device (2) comprises a rear face (24) which faces the body of the user in a mobile wearing position, and a front face (28) which is opposite said rear face and which faces away from the body of the user and forms a visible face (26) of the device (2), the visible face (26) of the device (2) being formed at least in part by a leg (44), in particular a leg plate, of the L-shaped housing part, and the rear face (24) being formed at least in part by a rear wall of the other housing part.

11. Device (2) according to any of the preceding claims, **characterized in that** at least one transparent region (14) is formed in at least one wall (12) of the second housing part (6), through which region it is possible to view the interior (16) of the container (10) when this transparent region (14) is not covered.

12. Device (2) according to claim 10, **characterized in that** the visible face (26) and/or the rear face (24) of the device (2) is made of a non-transparent material and, when the housing parts are joined as intended, the leg plate (44) of the first housing part (4) covers at least 90% or preferably all of the second housing part (6) in a viewing direction from the front onto the visible face (26) of the device (2) that faces away from the body.

13. Device (2) according to one or more of the preceding claims, **characterized in that** the device (2) has a beltlike holding means (8), such that it can be worn on the body of the user and carried around.

14. Device (2) according to claim 13, **characterized in that** the holding means (8) can be releasably fixed to the device (2) such that the holding means (8), in an operating position, covers a transparent region (14) of a wall (12) of the second housing part (6), through which region it is possible to view the interior (16) of the container (10) when the holding means (8) is not in the operating position.

15. Device (2) according to claim 13 or claim 14, **characterized in that** the holding means (8), in the operating position, at least partially surrounds a peripheral face (30) arranged between the rear face (24) and the front face (28) of the device (2), such that the peripheral face (30) of the device (2) is at least partially covered by the holding means (8).

## Revendications

1. Dispositif (2) destiné à fournir de la pression négative pour le traitement par pression négative de plaies sur le corps humain, avec un dispositif générateur de pression négative et un récipient (10) destiné à recevoir des liquides dans un intérieur (16) du récipient (10), en particulier des sécrétions de plaie aspirées au niveau d'une plaie, avec un raccord (16) pour une conduite d'aspiration (22) menant vers le corps, dans lequel le dispositif générateur de pression négative est agencé dans une première partie de boîtier (4) du dispositif (2) et le récipient (10) est formé par une deuxième partie de boîtier (6) du dispositif (2), dans lequel la deuxième partie de boîtier (6) du dispositif (2) peut être fixée à la première partie de boîtier (4) et peut être détachée manuellement, et, à l'état fixé, l'intérieur (16) du récipient (10) peut être alimenté en pression négative via un raccord (54b) par le dispositif générateur de pression négative, de sorte qu'une communication de pression négative peut être établie entre le dispositif générateur de pression négative, le récipient (10) et la conduite d'aspiration (22) menant vers le corps,
dans lequel au moins une partie de boîtier (4, 6), vue de côté, est réalisée pour l'essentiel en forme de L, **caractérisé par le fait que** la partie de boîtier en forme de L forme un guidage de siège coulissant pour l'autre partie de boîtier de sorte que les parties de boîtier (4, 6) peuvent être mises l'une contre l'autre de manière translationnelle dans un état assemblé comme prévu, et que sur une face intérieure (42) d'une branche (44) de la partie de boîtier en forme de L est réalisé au moins un premier moyen de guidage coulissant (46a) et que l'autre partie de boîtier comprend un deuxième moyen de guidage coulissant (46b) qui est complémentaire du premier moyen de guidage coulissant (46a) de sorte que l'autre partie de boîtier peut être poussée sur la branche (44) de la partie de boîtier en forme de L tout en étant guidée de manière translationnelle, dans lequel lesdits moyens de guidage coulissant (46a, 46b) s'engagent l'un dans l'autre, et que les premier et deuxième moyens de guidage coulissant (46a, 46b) comprennent au moins un rail de guidage et au moins une rainure de guidage complémentaire de celui-ci.

2. Dispositif (2) selon la revendication 1, **caractérisé par le fait que** la branche (44) de la partie de boîtier en forme de L est formée par une plaque de branche qui présente en particulier une épaisseur transversale à son extension surfacique, qui est de 10 mm tout au plus, en particulier de 8 mm tout au plus, en particulier de 5 mm tout au plus, en particulier de 3 mm tout au plus.

3. Dispositif (2) selon la revendication 1 ou 2, **caractérisé par le fait que**, à l'état assemblé comme prévu, la deuxième partie de boîtier (6) est bord à bord avec la première partie de boîtier (4).

4. Dispositif (2) selon une ou plusieurs des revendications précédentes, **caractérisé par le fait que**, vue de côté, la première partie de boîtier (4) est réalisée pour l'essentiel en forme de L et que la deuxième partie de boîtier (6) présente un profil qui complète de manière complémentaire la première partie de boîtier (4) de sorte que le dispositif (2) présente pour l'essentiel une forme de disque lorsque les parties de boîtier (4, 6) sont à l'état assemblé comme prévu.

5. Dispositif (2) selon une ou plusieurs des revendications précédentes, **caractérisé par le fait que** le dispositif (2) présente au moins un moyen de verrouillage (58) qui agit par encliquetage, enclenchement ou d'une autre manière en s'engageant derrière et au moyen duquel la deuxième partie de boîtier (6) peut être fixée de manière amovible contre la première partie de boîtier (4).

6. Dispositif (2) selon la revendication 5, **caractérisé par le fait que** ledit au moins un moyen de verrouillage (58) comprend une patte de verrouillage (60b) apte à être déviée à retour élastique et un élément (60a) qui est complémentaire de celle-ci et derrière lequel elle peut s'engager.

7. Dispositif (2) selon une ou plusieurs des revendications précédentes, **caractérisé par le fait que** dans une zone d'extrémité (62a, 62b) de premier et deuxième moyens de guidage coulissant (46a, 46b) des première et deuxième parties de boîtier (4, 6) sont réalisés des moyens de verrouillage (64a, 64b) agissant de concert les uns avec les autres.

8. Dispositif (2) selon la revendication 7, **caractérisé par le fait que** les moyens de verrouillage (64a, 64b) forment une prise de derrière transversalement à une direction de coulissement translationnelle (48).

9. Dispositif (2) selon une ou plusieurs des revendications précédentes, **caractérisé par le fait que**, en particulier dans une position de port mobile, le dispositif (2) peut être porté sur le corps d'un utilisateur et on peut l'avoir avec soi et que, à l'état assemblé comme prévu, vues en position de port mobile, les première et deuxième parties de boîtier (4, 6) du dispositif (2) sont disposées verticalement l'une au-dessus de l'autre de sorte qu'un plan de séparation (50) pour l'essentiel horizontal est formé entre les parties de boîtier (4, 6), dans lequel une partie supérieure du dispositif (2) est formée par la première partie de boîtier (4) comprenant le dispositif générateur de pression négative et une partie inférieure du dispositif est formée par la deuxième partie de boîtier (6) formant le récipient.

10. Dispositif (2) selon une ou plusieurs des revendications précédentes, **caractérisé par le fait que** le dispositif (2) présente une face arrière (24) qui est tournée vers le corps de l'utilisateur dans une position de port mobile ainsi qu'une face avant (28) qui est située en regard de celle-ci, montre dans la direction opposée au corps de l'utilisateur et forme une face visible (26) du dispositif (2), dans lequel la face visible (26) du dispositif (2) est formée au moins en partie par une branche (44), en particulier une plaque de branche, de la partie de boîtier en forme de L, et la face arrière (24) est formée au moins en partie par une paroi arrière de l'autre partie de boîtier.

11. Dispositif (2) selon l'une quelconque des revendications précédentes, **caractérisé par le fait que** dans au moins une paroi (12) de la deuxième partie de boîtier (6) est ménagée au moins une zone transparente (14) qui permet de voir dans l'intérieur (16) du récipient (10) lorsque cette zone transparente (14) n'est pas couverte.

12. Dispositif (2) selon la revendication 10, **caractérisé par le fait que** la face visible (26) et/ou la face arrière (24) du dispositif (2) est réalisée à partir d'un matériau non transparent, et que, à l'état assemblé comme prévu, la plaque de branche (44) de la première partie de boîtier (4) recouvre la deuxième partie de boîtier (6) à au moins 90 % ou de préférence complètement dans la direction de regard frontale sur la face visible (26) du dispositif (2), laquelle montre dans la direction opposée au corps.

13. Dispositif (2) selon une ou plusieurs des revendications précédentes, **caractérisé par le fait que** le dispositif (2) comprend un moyen de maintien (8) de type ceinture de sorte qu'il puisse être porté sur le corps de l'utilisateur et que l'on puisse l'avoir avec soi.

14. Dispositif (2) selon la revendication 13, **caractérisé par le fait que** le moyen de maintien (8) peut être fixé de manière amovible sur le dispositif (2) de telle sorte que, dans une position de fonctionnement, le moyen de maintien (8) recouvre une zone transparente (14) d'une paroi (12) de la deuxième partie de boîtier (6), laquelle permet de voir dans l'intérieur (16) du récipient (10) lorsque le moyen de maintien (8) n'est pas en position de fonctionnement.

15. Dispositif (2) selon la revendication 13 ou 14, **caractérisé par le fait que**, en position de fonctionnement, le moyen de maintien (8) entoure au moins en partie une face circonférentielle (30) disposée entre la face arrière (24) et la face avant (28) du dispositif (2) de sorte que la face circonférentielle (30) du dispositif (2) est recouverte au moins en partie par le moyen de maintien (8).
